# EUROPEAN PATENT APPLICATION

(11) **EP 4 624 583 A1**
(43) Date of publication of application: **01.10.2025**
(21) Application number: 22966076.6
(22) Date of filing: 22.11.2022
(51) Int. Cl.: C12N 15/85

(54) **REAGENT TEST KIT AND APPLICATION THEREOF IN SEQUENCING**

(71) Applicant: MGI Tech Co., Ltd., Shenzhen, Guangdong 518083 (CN)
(72) Inventor: JIA, Man, Shenzhen, Guangdong 518083 (CN); JIANG, Lan, Shenzhen, Guangdong 518083 (CN); LU, Zhenhao, Shenzhen, Guangdong 518083 (CN); TANG, Xintong, Shenzhen, Guangdong 518083 (CN); ZHANG, Feng, Shenzhen, Guangdong 518083 (CN); ZHENG, Min, Shenzhen, Guangdong 518083 (CN); XU, Chongjun, Shenzhen, Guangdong 518083 (CN)
(74) Representative: Potter Clarkson
(86) International application number: PCT/CN2022/133421
(87) International publication number: WO 2024/108376

(57) **Abstract**

Provided are a reagent test kit and use thereof in sequencing. The reagent test kit includes a first reagent and a second reagent. The first reagent is selected from a disulfide bond reducing agent. The second reagent is selected from a blocking reagent suited to blocking a disulfide bond.

## Description

### FIELD

The present disclosure relates to the biological field, and in particular, to a kit and use thereof in sequencing.

### BACKGROUND

Multiple displacement amplification (MDA) is an isothermal strand displacement amplification. Under constant temperature conditions, multiple random primers are annealed to the template, followed by a strand displacement reaction catalyzed by a DNA polymerase with strand displacement activity. The displaced single strands can randomly bind to the primers, be annealed, and extend, ultimately resulting in branch amplification. This technique relies on the unique strand displacement activity of the DNA polymerase. MDA has found widespread application in sequencing. The main steps of the process include: sequencing the first strand; after the first round of sequencing is complete, removing the template strand through multiple displacement amplification to form a complementary strand for the second round of sequencing; removing the DNA polymerase with strand displacement activity and the template strand through elution; and performing the second round of sequencing on the complementary strand.

After completing the MDA, the DNA polymerase with strand displacement activity must be removed through elution. However, the residual DNA polymerase may persist during the elution process. This residual polymerase can become entangled with dNTPs (chemically modified deoxyribonucleotides) at the beginning of complementary strand sequencing. Since the molecular structure of the modified dNTPs contains fluorescent dyes, the non-specific binding of enzymes to dNTPs due to the entanglement may lead to an increase in the background signal during sequencing, reducing the signal-to-noise ratio and ultimately compromising sequencing quality. Therefore, it is crucial to identify reagents that can effectively remove DNA polymerase with strand displacement activity to prevent its interference with complementary strand sequencing.

### SUMMARY

The present disclosure aims to solve the technical problems existing in the prior art at least to a certain extent.

It should be noted that the present disclosure is completed based on the following findings of the inventors.

The inventors have found that by disrupting the three-dimensional structure of DNA polymerase, the entanglement between the enzyme and the modified dNTPs containing fluorescent dyes can be avoided. Since the enzyme's three-dimensional structure is primarily stabilized by disulfide bonds, the inventors innovatively first use a disulfide-reducing agent during the elution process to break these disulfide bonds in the enzyme structure, and then use a blocking reagent to block thiol groups, thereby allowing the dNTPs to be released and reducing the background signal. Additionally, the inventors found that the residual DNA polymerase during the elution process is mainly due to hydrogen bonds between the enzyme and the modified chip. By using a protonating agent during the elution process, the hydrogen bonds can be broken, facilitating the removal of DNA polymerase, preventing its residue from affecting subsequent sequencing. In this way, the sequencing quality can be improved. In the meantime, the amount of elution reagent required can be reduced, and the elution time can be shortened, thereby improving sequencing efficiency.

To this end, in one aspect of the present disclosure, the present disclosure provides a kit. According to an embodiment of the present disclosure, the kit includes: a first reagent selected from a disulfide-reducing agent, and a second reagent selected from a blocking reagent suitable for blocking a disulfide bond.

According to an embodiment of the present disclosure, in the kit, the disulfide bonds in the DNA polymerase are broken by the disulfide-reducing agent, and then the thiol groups are blocked by the blocking reagent, thereby preventing entanglement with the modified dNTPs containing fluorescent dyes during the sequencing process. In this way, an increase in background signal and a decrease in the signal-to-noise ratio can be avoided, thereby improving sequencing quality.

According to an embodiment of the present disclosure, the kit further includes a third reagent, and the third reagent includes a protonating agent.

According to an embodiment of the present disclosure, the protonating agent is selected from ammonium salts. The inventors found that ammonium salts have a strong ability to break the hydrogen bonds between the DNA polymerase and the modified chip, facilitating the removal of the DNA polymerase. Additionally, ammonium salts can be easily removed through elution and are unlikely to have any adverse effects on subsequent sequencing.

According to an embodiment of the present disclosure, the protonating agent is selected from the group consisting of ammonium chloride, ammonium nitrate, ammonium sulfate, and combinations thereof. Thus, the hydrogen bonds between the DNA polymerase and the modified chip can be effectively broken, facilitating the removal of the DNA polymerase. Additionally, these reagents can be easily removed through elution and are unlikely to have any adverse effects on subsequent sequencing. Among these reagents, ammonium sulfate provides the most effective result.

In another aspect of the present disclosure, the present disclosure provides use of the above-mentioned kit in sequencing. Thus, the use of the kit according to an embodiment of the present disclosure effectively ensures sequencing quality, reduces the amount of elution reagent required in the sequencing reaction process, shortens the elution time, and improves sequencing efficiency.

In yet another aspect of the present disclosure, the present disclosure provides a sequencing method using the above-mentioned kit. According to an embodiment of the present disclosure, the method includes: performing a multiple displacement amplification treatment on a template strand on a chip, to obtain a complementary strand, a DNA polymerase with strand displacement activity being used in the multiple displacement amplification; loading the first reagent onto the chip, and removing the first reagent through elution; loading the second reagent onto the chip, incubating, and removing the second reagent through elution; and performing a sequencing reaction on the template strand or the complementary strand. Thus, the use of the method according to an embodiment of the present disclosure can obtain high-quality sequencing data, reduces the amount of elution reagent required in the sequencing reaction process, shortens the elution time, and improves sequencing efficiency.

Additional aspects and advantages of the present disclosure will be partially outlined in the following description, while others will be apparent from the description or can be learned through the practice of the disclosure.

### BRIEF DESCRIPTION OF THE DRAWINGS

The above and/or additional aspects and advantages of the present disclosure will be apparent and easily understood from the description of the embodiments in conjunction with the following drawings, in which:
FIG. 1 shows a schematic flowchart of a sequencing method using a kit according to an embodiment of the present disclosure.
FIG. 2 shows a schematic diagram of Q30 value analysis for double-stranded DNA according to an embodiment of the present disclosure.
FIG. 3 shows a schematic diagram of mismatch rate analysis after comparing DNA sequencing data with a standard gene reference, according to an embodiment of the present disclosure.

### DETAILED DESCRIPTION

The embodiments of the present disclosure are described in detail below. The embodiments described below are exemplary and are only used to explain the present disclosure, and should not be construed as limiting the present disclosure.

The present disclosure provides a kit, use of the kit in sequencing, and a sequencing method using the kit, which will be described in detail below.

### Kit

In one aspect of the present disclosure, the present disclosure provides a kit. According to an embodiment of the present disclosure, the kit includes a first reagent selected from a disulfide-reducing agent, and a second reagent selected from a blocking reagent suitable for blocking a disulfide bond.

In the kit according to an embodiment of the present disclosure, the disulfide-reducing agent breaks the disulfide bonds in the DNA polymerase. Subsequently, the thiol groups are blocked by the blocking reagent, thereby preventing entanglement with the modified dNTPs containing fluorescent dyes during the sequencing process. Thus, an increase in background signal and a decrease in the signal-to-noise ratio can be prevented, thereby improving sequencing quality.

According to an embodiment of the present disclosure, the disulfide-reducing agent is selected from the group consisting of β-mercaptoethanol, dithiothreitol, tris(2-carboxyethyl) phosphine, and combinations thereof. The inventors identified the above preferred disulfide-reducing agent through extensive experimentation and found that they can effectively break the disulfide bonds. Additionally, the above-mentioned reagents can be easily removed through elution and are unlikely to have any adverse effects on subsequent sequencing. Among these reagents, dithiothreitol (DTT) provides the most effective result.

According to an embodiment of the present disclosure, the blocking reagent is a reagent that irreversibly blocks a thiol group. By selecting the irreversible blocking reagent, the blocked thiol group can be prevented from being reversibly restored to a disulfide bond during the sequencing process, thereby ensuring sequencing quality.

According to an embodiment of the present disclosure, the blocking reagent is selected from the group consisting of N-ethylmaleimide, iodoacetamide, and combinations thereof. The inventors identified the preferred blocking reagents through extensive experimentation and found that they can effectively block the thiol group, thereby preventing the interference with complementary strand sequencing. Additionally, these reagents can be easily removed through elution and are unlikely to have any adverse effects on subsequent sequencing. While iodoacetamide is prone to decomposition by light, which slightly compromises its stability, it can still be used in a light-proof environment. In contrast, N-ethylmaleimide exhibits good stability and is less susceptible to environmental factors.

According to an embodiment of the present disclosure, the kit further includes a third reagent. The third reagent includes a protonating agent.

The term "protonating agent" used in the present disclosure refers to an agent that can donate protons (H) to atoms, molecules, or ions. Before sequencing using a sequencing chip, the sequencing chip is pre-modified. The inventors found that residual DNA polymerase during the elution process is primarily attributed to hydrogen bonds between the enzyme and the modified chip. These hydrogen bonds can be broken by using the protonating agent during the elution process, facilitating the removal of the DNA polymerase. The first, second, and third reagents of the present disclosure are used in combination to function synergistically to mitigate the adverse effects of residual DNA polymerase on complementary strand sequencing, thereby improving sequencing quality.

According to an embodiment of the present disclosure, the protonating agent is selected from ammonium salts. The inventors found that ammonium salts have a strong ability to break the hydrogen bonds between the DNA polymerase and the modified chip, facilitating the removal of the DNA polymerase. Additionally, ammonium salts can be easily removed through elution and are unlikely to have any adverse effects on subsequent sequencing.

According to an embodiment of the present disclosure, the protonating agent is selected from the group consisting of ammonium chloride, ammonium nitrate, ammonium sulfate, and combinations thereof. Thus, the hydrogen bonds between the DNA polymerase and the modified chip can be effectively broken, facilitating the removal of the DNA polymerase. Additionally, these reagents can be easily removed through elution and are unlikely to have any adverse effects on subsequent sequencing. Among the reagents, ammonium sulfate provides the most effective result.

According to an embodiment of the present disclosure, the third reagent further includes a buffer solution; a concentration of the protonating agent in the third reagent ranges from 50 to 80 mM, and a pH value of the third reagent ranges from 6 to 7. Thus, the background signal can be better removed. Specifically, the buffer solution used may be WB1 buffer. The source of the WB1 buffer is not strictly limited, and it may either be prepared in-house or obtained commercially.

According to an embodiment of the present disclosure, the first reagent and the second reagent are provided in independent forms.

According to an embodiment of the present disclosure, the first reagent, the second reagent, and the third reagent are provided in independent forms.

According to an embodiment of the present disclosure, the kit further includes a DNA polymerase and/or a polymerization reaction reagent. According to an embodiment of the present disclosure, the DNA polymerase is a DNA polymerase with strand displacement activity, such as a phi29 DNA polymerase. The DNA polymerase with strand displacement activity, particularly phi29 DNA polymerase having a disulfide bond. The above-mentioned disulfide-reducing agent can be used to break the disulfide bond in the DNA polymerase, and then the thiol group can be blocked by the blocking reagent. In this way, the entanglement of modified dNTPs containing fluorescent dyes during sequencing, which could otherwise lead to increased background signals and a decreased signal-to-noise ratio, can be avoided. Additionally, the hydrogen bonds can be broken by using the protonating agent during the elution process, facilitating the removal of the DNA polymerase. The first, second, and third reagents of the present disclosure are used in combination to function synergistically to mitigate the adverse effects of residual DNA polymerase on complementary strand sequencing, thereby improving sequencing quality

The term "polymerization reaction reagent" used in the present disclosure refers to a reagent capable of participating in a polymerization reaction. The polymerization reaction reagent is not specifically limited in the present disclosure, and it can include any reagent commonly used in the art for multiple displacement amplification treatments, sequencing reactions, or similar applications.

### Use of the Kit in Sequencing

In another aspect of the present disclosure, the use of the above-mentioned kit in sequencing is provided. By utilizing the kit according to an embodiment of the present disclosure, the quality of sequencing can be effectively ensured.

According to an embodiment of the present disclosure, the sequencing includes performing multiple displacement amplification using a DNA polymerase with strand displacement activity.

It should be noted that the features and advantages described above for the kit also apply to this use and will not be repeated here.

### Sequencing Method Using the Kit

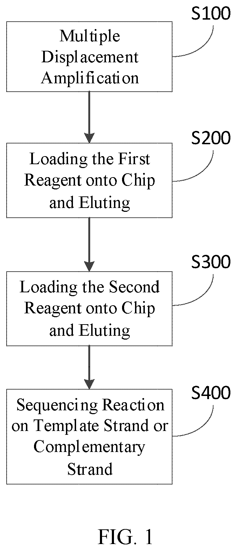

In yet another aspect of the present disclosure, a sequencing method using the above kit is provided. According to an embodiment of the present disclosure, as illustrated in FIG. 1, the method includes the following steps.

S100: Multiple Displacement Amplification

In this embodiment, the multiple displacement amplification treatment is performed on a template strand on a chip to obtain a complementary strand. A DNA polymerase with strand displacement activity is used in the multiple displacement amplification.

According to an embodiment of the present disclosure, the DNA polymerase with strand displacement activity is phi29 DNA polymerase. Phi29 DNA polymerase is a mesophilic DNA polymerase cloned from the *Bacillus subtilis* bacteriophage phi29. This enzyme is characterized by its ability of unwinding, removing the leading strand in the enzyme's forward direction from the original template strand and performing displacement to obtain the complementary strand. The specific steps of the multiple displacement amplification process are not specifically limited in the present disclosure, and conventional techniques in the art can be used, as long as phi29 DNA polymerase is employed in the process.

In this embodiment, the chip is subjected to elution.

S200: the first reagent is loaded onto chip, and the chip is subjected to elution.

In this embodiment, the first reagent is loaded onto the chip and then removed through elution, thereby disrupting the disulfide bonds of any residual DNA polymerase, facilitating the subsequent blocking of the disulfide bonds by means of the blocking reagent.

According to an embodiment of the present disclosure, before loading the first reagent onto the chip, the chip that has subjected to the multiple displacement amplification treatment is cleaned. A third reagent is used in the cleaning. Before sequencing, the sequencing chip is pre-modified. The inventors found that residual DNA polymerase during the cleaning process is primarily attributed to hydrogen bonds between the enzyme and the modified chip. These hydrogen bonds can be broken by using the protonating agent during the elution process, facilitating the removal of the DNA polymerase.

According to an embodiment of the present disclosure, the cleaning includes: loading the third reagent onto the eluted chip, incubating for 2 to 5 minutes, and then removing the third reagent by eluting with an elution reagent. The incubation allows the third reagent to effectively break the hydrogen bonds, preventing the retention of DNA polymerase. Specifically, the elution reagent used for this step is the same as that used for the elution in step S200.

According to an embodiment of the present disclosure, before the cleaning, the chip that has subjected to the multiple displacement amplification treatment is treated through elution. Specifically, the elution reagent used in the elution is derived from the MGISEQ-2000RS sequencer kit. Thus, both the template strand and the DNA polymerase are effectively eluted, with the composition of the eluent having no adverse effect on subsequent sequencing.

S300: the second reagent is loaded onto chip, and the chip is subjected to elution.

In this embodiment, the second reagent is loaded onto the chip, incubated, and removed through elution, thereby blocking the previously broken disulfide bonds.

According to an embodiment of the present disclosure, the incubation is performed for 20 to 40 minutes. Thus, all thiol groups are irreversibly blocked to prevent any adverse effects on subsequent second-strand sequencing.

S400: a sequencing reaction is performed on the template strand or the complementary strand.

In this step, the template strand or the complementary strand is subjected to a sequencing reaction.

It should be noted that the order of steps S100 and S500 is not strictly limited in the present disclosure. Specifically, the timing of the sequencing reaction of the template strand is flexible, as long as the sequencing of the template strand can be implemented. For example, the template strand may be sequenced first, followed by multiple displacement amplification to generate the complementary strand, the chip is then subjected to the treatment in steps S200 and S300, and then the sequencing is performed on the complementary strand. Alternatively, the template strand may be first subjected to the multiple displacement amplification to generate the complementary strand, the chip is then subjected to the treatment in steps S200 and S300, and then the sequencing is then performed on both the template and complementary strands. According to an embodiment, the sequencing method further includes, prior to said performing the multiple displacement amplification treatment to obtain a complementary strand: performing a sequencing reaction on the template strand fixed on the chip. According to an embodiment, the sequencing method further includes: performing the multiple displacement amplification treatment on the template strand fixed onto the chip to obtain a complementary strand, treating the chip with the first reagent and the second reagent, and performing the sequencing reaction on the template strand and the complementary strand.

According to an embodiment of the present disclosure, the sequencing is performed on a DNBSEQ sequencing platform.

It should be noted that the features and advantages described above for the kit also apply to the sequencing method using the kit and will not be repeated here.

The scheme of the present disclosure will be illustrated below in conjunction with the examples. It will be appreciated by those skilled in the art that the following examples are only used to illustrate the present disclosure and should not be considered as limiting the scope of the present disclosure. Where specific techniques or conditions are not indicated in the examples, the techniques or conditions described in the literature in the art or the product specifications are used. The reagents or instruments used, not indicated by the manufacturer, are all conventional products that can be obtained commercially.

### Example 1

### 1. Experimental Equipment

MGISEQ-2000RS sequencer, MGIDL-200H loader, MGISEQ-2000RS sequencing carrier were used. The excitation wavelengths for the MGISEQ-2000RS sequencer were 532 nm and 650 nm, respectively.

### 2. Reagents and Raw Materials Used in the Experiments

Ammonium bicarbonate (analytical grade), N-ethylmaleimide (analytical grade), ammonium sulfate (analytical grade), elution reagent (from MGISEQ-2000RS sequencer kit), WB1 buffer (from BGI), and ultrapure water were used.

The experiments were conducted using *Escherichia coli* single-stranded circular DNA (i.e., standard library reagent V3.0) as the template. The CoolMPS high-throughput sequencing reagent kit (MGISEQ-2000RS FCL PE100, containing phi29 DNA polymerase) was employed to prepare DNA nanoballs, which were subsequently loaded onto the chip for sequencing.

dNTP Mixture: containing dATP, dTTP, dCTP and dGTP, each with fluorescent modifications.

### 3. Experimental Methods and Procedures

### (a) Preparation of Blocking Reagent:

Ammonium bicarbonate was dissolved in ultrapure water to prepare an ammonium bicarbonate aqueous solution. N-ethylmaleimide (NEM) was then dissolved in the ammonium bicarbonate aqueous solution to obtain a blocking reagent.

### (b) Preparation of 62.5 mM Slide Wash Reagent (pH 6.45)

Ammonium sulfate powder was accurately weighed and dissolved in ultrapure water to prepare an ammonium sulfate aqueous solution (1M). Such an ammonium sulfate aqueous solution (1M) was then mixed with WB1 buffer to prepare a Slide wash reagent (62.5 mM), the pH of which was adjusted to 6.45 using concentrated sulfuric acid (H₂SO₄).

### (c) DNA Sequencing Methods

Step 1: the DNA nanoballs were loaded onto the prepared chip.

Step 2: the prepared dNTP mixture was pumped into the chip, and the first-strand sequencing was performed according to the instructions provided by the CoolMPS high-throughput sequencing reagent kit.

Step 3: After the first strand was sequenced, a multiple displacement amplification was performed using phi29 DNA polymerase to generate the second strand. Once the multiple displacement amplification treatment was complete, excess phi29 DNA polymerases were washed away using an elution reagent derived from the CoolMPS high-throughput sequencing reagent kit.

Step 4: the following experimental groups were established and subjected to the corresponding operations, respectively.

Control Group: WB1 buffer (pH adjusted to 6.45 with concentrated H₂SO₄) was pumped into the chip to react for 3 minutes, followed by elution with WB1 buffer for second-strand sequencing.

Experimental Group: Slide wash reagent (62.5 mM, pH 6.45) was pumped into the chip to react for 3 minutes, followed by elution with Slide wash reagent (62.5 mM, pH 6.45) for second-strand sequencing.

Experimental Group 1: Slide wash reagent (62.5 mM, pH 6.45) was pumped into the chip to react for 3 minutes, followed by elution with Slide wash reagent (62.5 mM, pH 6.45). Then, DTT was pumped into the chip to break the disulfide bonds in the enzyme protein, and then washed away by eluting with the elution reagent. The blocking reagent was pumped into the chip and incubated for 30 minutes, and then the solution was washed away by eluting with the elution reagent before second-strand sequencing.

Experimental Group 2: Slide wash reagent (62.5 mM, pH 6.45) was pumped into the chip to react for 3 minutes, followed by elution with Slide wash reagent (62.5 mM, pH 6.45). Then, DTT was pumped into the chip to break the disulfide bonds in the enzyme protein, and then washed away by eluting with the elution reagent. The blocking reagent was pumped into the chip and incubated for 30 minutes, and then the solution was washed away by eluting with the elution reagent before second-strand sequencing. In order to validate that this processing method was also compatible with sequencing kits using FT regeneration reagents, reagent No. 9 (regeneration reagent) from Experimental Group 1 was replaced with reagent No. 15 (FT regeneration reagent) from the DNBSEQ-G99RS high-throughput sequencing reagent kit (G99 SM FCL PE150, material code 940-000410-00). The experiments were then performed, with all other operations and reagents unchanged.

### 4. Experimental Results

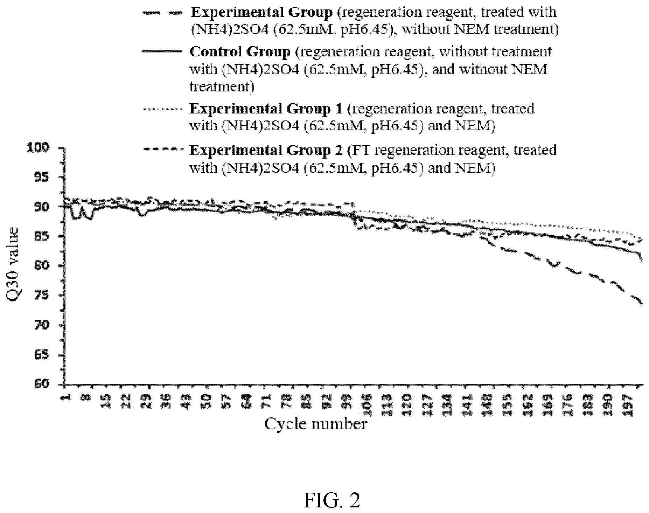

As shown in FIG. 2, compared to the Control Group in which the chip was not subjected to the treatments of disulfide bond opening, blocking, or protonating agent treatment, the sequencing results of PE100, in which (NH₄)₂SO₄ was used as a protonating agent to clean the chip, DTT was used to open the disulfide bonds, and NEM was employed as a blocking reagent to block the thiol groups, exhibited a higher Q30 value and a lower rate of Q30 decrease. Such a result demonstrates that the present disclosure is applicable to various sequencing and regeneration reagents, confirming its universality and suitability for a wide range of applications.

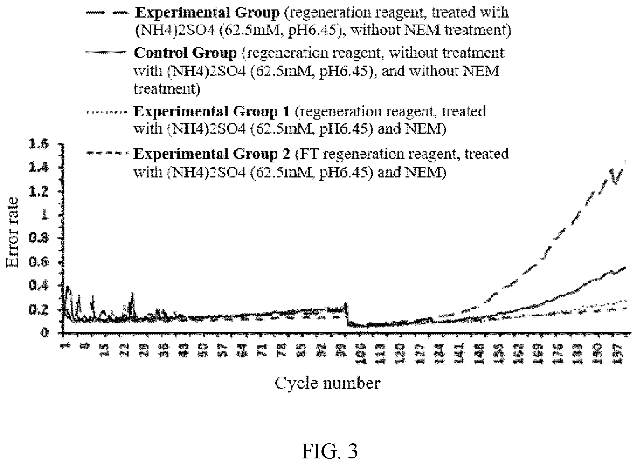

As shown in FIG. 3, compared to the Control Group in which the chip was not subjected to the treatments of disulfide bond opening, blocking, or protonating agent treatment, the sequencing results of PE100, in which (NH₄)₂SO₄ was used as a protonating agent to clean the chip, DTT was used to open the disulfide bonds, and NEM was employed as a blocking reagent to block the thiol groups, exhibited a lower error rate. Such a result demonstrates that the present disclosure is applicable to various sequencing and regeneration reagents, confirming its universality and suitability for a wide range of applications.

In the specification, the description with reference to the term "an embodiment", "some embodiments", "example", "specific example", or "some examples" means that the specific features, structures, materials or characteristics described in conjunction with the embodiment or example are included in at least an embodiment or example of the present disclosure. In the specification, the schematic representations of the above terms do not necessarily refer to the same embodiment or example. Moreover, the specific features, structures, materials or characteristics described may be combined in any one or more embodiments or examples in a suitable manner. In addition, those skilled in the art may combine and incorporate the different embodiments or examples described in this specification and the features of the different embodiments or examples, without contradiction.

Although the embodiments of the present disclosure have been shown and described above, it is to be understood that the above embodiments are exemplary and are not to be construed as limitations of the present disclosure. A person skilled in the art may change, modify, replace and vary the above embodiments within the scope of the present disclosure.

## Claims

1. A kit, comprising:
a first reagent selected from a disulfide-reducing agent; and
a second reagent selected from a blocking reagent suitable for blocking a disulfide bond.

2. The kit according to claim 1, wherein the disulfide-reducing agent is selected from the group consisting of β-mercaptoethanol, dithiothreitol, tris(2-carboxyethyl) phosphine, and combinations thereof.

3. The kit according to claim 1, wherein the disulfide-reducing agent is dithiothreitol.

4. The kit according to claim 1, wherein the blocking reagent is a reagent capable of irreversibly blocking a thiol group.

5. The kit according to claim 1, wherein the blocking reagent is selected from the group consisting of N-ethylmaleimide, iodoacetamide, and a combination thereof.

6. The kit according to claim 1, wherein the blocking reagent is N-ethylmaleimide.

7. The kit according to claim 1, further comprising a third reagent, wherein the third reagent comprises a protonating agent.

8. The kit according to claim 7, wherein the protonating agent is selected from ammonium salts.

9. The kit according to claim 7, wherein the protonating agent is selected from the group consisting of ammonium chloride, ammonium nitrate, ammonium sulfate, and combinations thereof.

10. The kit according to claim 7, wherein the protonating agent is ammonium sulfate.

11. The kit according to claim 7, wherein:
the third reagent further comprises a buffer solution; and
a concentration of the protonating agent in the third reagent ranges from 50 mM to 80 mM, and a pH value of the third reagent ranges from 6 to 7.

12. The kit according to claim 1, wherein the first reagent and the second reagent are provided in independent forms.

13. The kit according to claim 7, wherein the first reagent, the second reagent, and the third reagent are provided in independent forms.

14. The kit according to claim 1, further comprising a DNA polymerase and/or a polymerization reaction reagent.

15. The kit according to claim 14, wherein the DNA polymerase is selected from a DNA polymerase with strand displacement activity.

16. Use of the kit according to any one of claims 1 to 15 in sequencing.

17. The use according to claim 16, wherein the sequencing comprises performing multiple displacement amplification using a DNA polymerase with strand displacement activity.

18. A sequencing method using the kit according to any one of claims 1 to 15, the method comprising:
performing a multiple displacement amplification treatment on a template strand on a chip, to obtain a complementary strand, wherein a DNA polymerase with strand displacement activity is used in the multiple displacement amplification treatment;
loading the first reagent onto the chip, and removing the first reagent through elution;
loading the second reagent onto the chip, incubating, and removing the second reagent through elution; and
performing a sequencing reaction on the template strand or the complementary strand.

19. The method according to claim 18, wherein a duration of said incubating ranges from 20 minutes to 40 minutes.

20. The method according to claim 18, further comprising, subsequent to said performing the multiple displacement amplification treatment and prior to said loading the first reagent onto the chip:
cleaning the chip using a third reagent.

21. The method according to claim 20, wherein said cleaning comprises:
loading, subsequent to said performing the multiple displacement amplification treatment, the third reagent onto the chip;
incubating for 2 to 5 minutes; and
removing the third reagent through elution with an elution reagent.

22. The method according to claim 18, further comprising, prior to said performing the multiple displacement amplification treatment to obtain a complementary strand:
performing a sequencing reaction on the template strand fixed on the chip.

23. The method according to claim 18, further comprising:
performing the multiple displacement amplification treatment on the template strand fixed on the chip to obtain a complementary strand;
treating the chip with the first reagent and the second reagent; and
performing the sequencing reaction on the template strand and the complementary strand.
